# EUROPEAN PATENT SPECIFICATION

(11) **EP 4 396 700 B1**
(45) Date of publication and mention of the grant of the patent: **30.07.2025**
(21) Application number: 22768367.9
(22) Date of filing: 19.08.2022
(51) Int. Cl.: G06F 18/00, G06T 5/00, G06V 30/164

(54) **DENOISING OF MEDICAL IMAGES USING A MACHINE-LEARNING METHOD**
ENTRAUSCHEN VON MEDIZINISCHEN BILDERN UNTER VERWENDUNG EINES MASCHINENLERNVERFAHRENS
DÉBRUITAGE D'IMAGES MÉDICALES À L'AIDE D'UN PROCÉDÉ D'APPRENTISSAGE AUTOMATIQUE

(30) Priority: 31.08.2021 US 202163238918 P
(43) Date of publication of application: 10.07.2024
(73) Proprietor: Koninklijke Philips N.V., 5656 AG Eindhoven (NL)
(72) Inventor: WUELKER, Christian, 5656 AG Eindhoven (NL); SCHNELLBAECHER, Nikolas, David, 5656 AG Eindhoven (NL); BERGNER, Frank, 5656 AG Eindhoven (NL); BROWN, Kevin, Martin, 5656 AG Eindhoven (NL); GRASS, Michael, 5656AG Eindhoven (NL)
(74) Representative: Philips Intellectual Property & Standards
(86) International application number: PCT/EP2022/073205
(87) International publication number: WO 2023/030922

(56) References cited:
- EP-A2- 2 504 811
- EP-B1- 2 504 811
- US-A1- 2017 098 317
- US-A1- 2019 378 270
- JIFARA WORKU ET AL: "Medical image denoising using convolutional neural network: a residual learning approach", THE JOURNAL OF SUPERCOMPUTING, SPRINGER US, NEW YORK, vol. 75, no. 2, 1 June 2017 (2017-06-01), pages 704 - 718, XP036715215, ISSN: 0920-8542, [retrieved on 20170601], DOI: 10.1007/S11227-017-2080-0

## Description

### FIELD OF THE INVENTION

The present invention relates to the field of medical imaging, and in particular, to the denoising of medical images.

### BACKGROUND OF THE INVENTION

Medical imaging is becoming of increasing interest in the medical profession in order to (non-invasively) aid the assessment and/or diagnosis of the condition of a subject or patient under investigation. Various forms of medical imaging approaches or modalities are known in the art, and may employ invasive or non-invasive imaging techniques. Examples include computed tomography (CT) or X-ray imaging, magnetic resonance (MR) imaging, (intravenous) ultrasound imaging, positron emission tomography PET imaging, optical coherence tomography, transesophageal echocardiography and so on.

An ongoing concern with medical images is noise or artefacts. When assessing the condition of a subject, noise or artefacts can hinder an identification of potentially important features of the subject (e.g. by obscuring them or rendering them difficult to identify), as well as potentially being mistaken for diagnostically relevant features. There is therefore an ongoing desire to reduce the amount of noise in medical images.

One recently developed approach to perform accurate denoising of medical images is to use an appropriately trained machine-learning method to perform the denoising. However, there are a large number of parameters that could be modified during a medical imaging process. For instance, in CT scanning, different forms of reconstruction filters could be used in the generation of a CT medical image. Machine-learning methods are prone to overfit to their training data, and thus often fail to generalize to the large range of real-world parameter settings (which may not always be sampled in training).

There is therefore a need for an improved approach for denoising medical images.

US 2019/378270 A1 describes a medical image diagnostic apparatus including processing circuity. The processing circuity inputs a noise correlation map and a medical image or an intermediate image to a learned model that is functioned to generate a denoise image.

### SUMMARY OF THE INVENTION

The invention is defined by the claims.

According to examples in accordance with an aspect of the invention, there is provided a computer-implemented method of denoising a medical image to thereby produce a denoised medical image.

The computer-implemented method comprises: obtaining the medical image formed of a plurality of pixels; obtaining a noise map containing an estimated measure of a statistical parameter for each pixel of the medical image; modifying the medical image using the noise map to produce a modified medical image; and processing the modified medical image, using a machine-leaning method, to produce the denoised medical image; wherein modifying the medical image comprises dividing the medical image by the noise map.

The proposed invention proposes an approach in which a medical image undergoes a global (i.e. image-wide) noise-level normalization/homogenization based on a statistical parameter of noise of the medical image. The normalized/homogenization medical image is then processed using a machine-learning method, e.g. to perform further noise reduction.

The machine-learning method thereby consistently processes medical images that have already been normalized or homogenized based on local/global conditions. This means that images provided to the machine-learning method are at consistent noise levels and/or homogenized (e.g. to decorrelate noise). The machine-learning method may thereby be trained on normalized/homogenized medical images, meaning that a problem of overfitting is avoided.

The proposed approach thereby provides an improved and more accurate method of denoising a medical image.

The division of the medical image by the noise map may be a pixel-wise or point-wise division.

In some examples, the step of processing the modified medical image comprises: processing the modified medical image using the machine-learning method to generate a predicted noise image, the predicted noise image representing a predicted amount of noise in each pixel of the modified medical image; multiplying the modified medical image by the noise map to produce a calibrated predicted noise image; and subtracting the calibrated predicted noise image or a scaled version of the calibrated predicted noise image from the medical image from the medical image to produce the denoised medical image.

In some examples, the step of processing the modified medical image comprises inputting the modified medical image to the machine-learning method and receiving, as output from the machine-learning method, the denoised medical image. The denoised medical image output by the machine-learning method may be uncalibrated (e.g. if the medical image is divided by the noise map to modify the medical image). Accordingly, the denoised medical image output by the machine-learning method may be recalibrated using the noise map. In particular, a reversal to (or inverse of) the modification performed on the medical image to produce the modified medical image may be applied to the denoised medical image to recalibrate it. This may comprise, for instance, (e.g. pixel-wise) multiplying the denoised medical image by the noise map to produce a recalibrated denoised medical image.

The machine-learning method may comprise or be a neural network. This provides an accurate and reliable approach for processing the modified medical image in order to perform the denoising process.

Preferably, the machine-learning method is a residual (output) machine-learning method, e.g. a residual neural network. In the context of the present disclosure, a residual machine-learning method is one that processes the modified medical image to provide a predicted noise image indicating a predicted amount of noise in each pixel of the modified medical image.

Use of a residual machine-learning method is advantageous, as it makes the output of the neural network more reliable. In particular, it can be assumed that a predicted noise image will be within a range or scope of outputs to which the machine-learning method has been trained (as noise has only a limited range of probable values). A non-residual or direct machine-learning method, e.g. one that attempts to directly predict a denoised medical image, is more likely to lead to a predicted denoised image that it outside of the range to which the machine-learning method was trained (i.e. is less reliable).

The noise map may provide an estimated amount of standard deviation or variance of noise for each pixel of the medical image.

In another example, the noise map provides, for each pixel of the medical image, an estimated correlation between the noise of the pixel and the noise of one or more neighboring pixels.

In yet another example, the medical image is one of a plurality of medical images, that represent a same scene, produced by a multi-channel imaging process; and the noise map provides, for each pixel of the medical image, an estimated measure of a covariance or correlation between the noise of that pixel and the noise of a corresponding pixel of another of the plurality of medical images.

This approach allows for crosstalk between channels of a multi-channel imaging process to be more effectively reduced or taken into account when denoising a medical image (of a multi-channel imaging process). It will be appreciated that the medical image of each channel, i.e. each of the plurality of images, may be separately processed using a herein described method.

In some examples, the step of obtaining the medical image comprises: obtaining a first medical image; processing the first medical image using a frequency filter to obtain a filtered medical image having values within a predetermined frequency range; and setting the filtered medical image as the medical image.

In at least one example, the method of further comprises: processing the first medical image to obtain a second filtered medical image having values within a second, different predetermined frequency range (e.g. not comprising any of the first frequency range); and combining the second filtered medical image and the denoised medical image to produce a denoised first medical image

In preferable examples, the medical image is a medical image that has been reconstructed from raw data using a first reconstruction algorithm; and the machine-learning method is a machine-learning method that has been trained using a training dataset that includes one or more training images that have been reconstructed from raw data using a second, different reconstruction algorithm. Such embodiments recognize that different reconstruction filters cause noise to have different characteristics. By homogenizing a medical image to be processed using the machine-learning method, a machine-learning method that has been trained using medical images produced with a different reconstruction filter can still be used with a high degree of reliability.

In some examples, the machine-learning method is one that has been trained using a training dataset that includes one or more training images that have each been modified with a respective noise map, preferably in an identical manner to the medical image to be processed using the machine-learning method. This approach increases an appropriateness and reliability of the machine-learning method.

The medical image may be a computed tomography medical image. The proposed approach has been identified as being particularly useful for medical images that can be produced using different reconstruction filters, and therefore is of particular use with computed tomography images for which a wide variety of reconstruction filters are available.

There is also proposed a computer program product comprising computer program code means which, when executed on a computing device having a processing system, cause the processing system to perform all of the steps of any herein described method.

There is also proposed a processing system configured to denoise a medical image to thereby produce a denoised medical image according to claim 13.

There is also proposed a system comprising: the processing system previously described; and a medical imaging system configured to generate the medical image and provide the generated medical image to the processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a flowchart illustrating a method according to an embodiment;
Figure 2 is a flowchart that illustrates a process for use in a method;
Figure 3 is a flowchart illustrating a method according to an embodiment;
Figure 4 illustrates an effect of a proposed approach;
Figure 5 illustrates a processing system according to an embodiment; and
Figure 6 illustrates a system according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an approach for denoising a medical image. A noise map, which defines an estimate of one or more statistical parameters for each pixel of the medical image, is used to modify or normalize the medical image. The modified medical image is then processed, using a machine-learning method, to produce a denoised medical image.

Embodiments are based on the realization that reconstruction filters or algorithms, used to produce a medical image, result in noise of the differently reconstructed medical images having different statistical properties. This makes denoising machine-learning methods less efficient, as they may have not been trained on images produced using the same reconstruction filter. By modifying the medical image using the estimates of a statistical parameter, each medical image may effectively have a normalized statistical level of noise. This facilitates greater consistency and therefore accuracy for a denoising machine-learning method.

The proposed concepts may be employed in any medical imaging system, which can be employed in a wide variety of clinical environments.

In the context of the present disclosure, a medical image is any image obtained using a medical imaging modality, such as an X-ray image, a CT (computed tomography) image, a PET (position emission tomography) image, an MR (magnetic resonance) image or an ultrasound image. Other forms of medical image will be apparent to the skilled person.

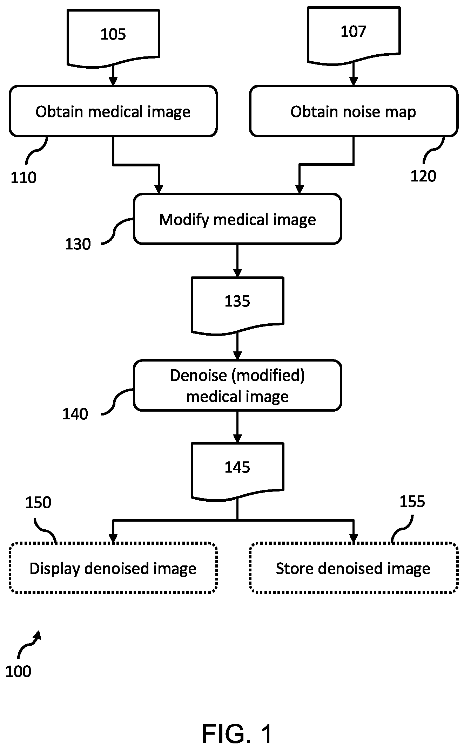

Figure 1 is a flowchart illustrating a method 100 for denoising a medical image 105 according to an embodiment.

Method 100 may be performed, for instance, on a single image. In another example, method 100 can be performed on one or more (e.g. each) of a plurality of images. The plurality of medical images may be produced by a multi-channel imaging process, so that each image represents a different channel, and may represent a same scene. In other words, each of the plurality of images may represent a same anatomical area.

The method 100 comprises a step 110 of obtaining the medical image to be denoised. The medical image is formed of a plurality of pixels, and may be two-dimensional (2D) or three-dimensional (3D). A pixel for a 3D image may be alternatively labelled a voxel.

Step 110 may itself comprise generating the medical image, e.g. using an appropriately configured medical imaging device. In other examples, step 110 may comprise obtaining an already generated medical image, e.g. from a medical imaging device and/or a memory or storage unit.

The method 100 further comprises a step 120 of obtaining a noise map 107 for the medical image. The noise map contains an estimated measure of a statistical parameter of noise for each pixel of the medical image. Put another way, the noise map defines, for each pixel of the medical image, an estimated value of a statistical parameter of noise for that pixel. It is emphasized that the noise map provides estimated statistical measures of noise, rather than of specific intensities at pixels of the medical image.

The noise map may contain a dedicated estimated measure for each pixel of the medical image (e.g. is formed of an equal number of pixels as the medical image). In other examples, the noise map may contain estimated measures for groups of two or more pixels, so that a single estimated measure represents a statistical parameter of noise for more than one pixel. Thus, a single estimated measure may represent a statistical parameter of noise for a respective region of the medical image.

In some examples, a noise map may contain an estimate of a noise variance and/or a noise standard deviation for each pixel of the medical image. Approaches for generating a noise map such as these are established in the art. A number of examples are disclosed in the United States Patent having patent number US 10,984,564 B2. Other examples include U.S. Pat. No. 9,1591,22 B2, filed Nov. 12, 2012, and entitled "Image domain de-noising," US 2016/0140725 A1, filed Jun. 26, 2014, and entitled, "Methods of utilizing image noise information," and U.S. Pat. No. 8,938,110 B2, filed Oct. 29, 2015, and entitled "Enhanced image data/dose reduction".

In another example, the noise map may contain (for each pixel/region) an estimated measure of correlation between the noise for said pixel/region and its neighboring pixels/regions. Any (coarse) method for denoising known in the art can be used to generate a (coarse) estimate of the noise in an image or image region. From such a noise estimate it is directly possible to estimate the local noise correlation by performing standard correlation analysis.

As yet another example, the noise map may contain a noise probability density function (noise PDF) for each pixel of the medical image or each region of the medical image. The noise PDF may be used, for instance, to determine or predict a noise variance and/or standard deviation of the pixel/region.

As yet another example, the noise map may contain a covariance or correlation between the noise of a pixel and the noise of a corresponding pixel of another medical image. The medical image and the other medical image may form part of a plurality of medical images that represent a same scene, e.g. and by produced by a multi-channel imaging process.

Step 120 may itself comprise a step of generating a noise map, e.g. using any previously described approach. Alternatively, step 120 may comprise obtaining an already generated noise map for the medical image, e.g. from a memory or storage unit.

The method 100 also performs a step 130 of modifying the medical image using the noise map to produce a modified medical image.

Step 130 may comprise, for instance, normalizing the medical image using the statistical measure of estimated noise. This effectively normalizes or aims to normalize this statistical measure across the entire medical image.

By way of example, step 130 may comprise dividing the medical image by the noise map. In particular, the value of each pixel of the medical image may be divided by the estimated measure of a statistical parameter of noise for that pixel, which is provided by the noise map. **In** this way, a point-wise or pixel-wise division of the medical image by an estimate of the statistical parameter of noise may be performed.

As another example, step 130 may comprise processing the noise map to get statistical information about the noise map and/or the medical image. This statistical information may then be used to modify the medical image.

For instance, the noise map may be processed to determine an average estimated deviation of the measure of a statistical parameter (e.g. across the entire image or for different sections of the image). The medical image may then be divided by the average estimated deviation, e.g. on a section-by-section basis for averages that represent an average for a particular section or across the whole image if the average indicates an average across the entire image.

As yet another example, the noise map may be processed to determine or predict a shape of the noise in the frequency domain. This shape may then be used to perform frequency-domain filtering of the medical image, e.g. to normalize the frequency of the noise in the medical image.

As another example, step 130 may comprise performing a decorrelation process on the noise of the medical image, i.e. decorrelating noise of the medical image. Thus, step 130 may comprise (spatially) decorrelating the noise of the medical image to produce the modified medical image.

This may be performed using a noise map that indicates a measure of correlation between different pixels and/or regions. As another example, this may be performed using a noise map that provides a noise PDF for each pixel and/or region, and processing the image using one or more means of approximate deconvolution or transformation of PDFs by letting the values run through some non-linear function

The skilled person would readily conceive of a wide variety of other approaches for modifying the medical image in step 130. More generally, step 130 is a step of modifying the medical image so that a noise response across the modified medical image is more homogenous than across the (original) medical image.

The output of step 130 is a modified medical image 135.

The method 100 then performs a process 140 of processing the modified medical image, using a machine-leaning method, to produce the denoised medical image.

Figure 1 illustrates one embodiment for performing process 140.

In this example, process 140 comprises directly predicting or inferring a denoised medical image 145 from the modified medical image 135. Thus, the machine-learning method may receive, as input, the modified medical image and provide, as output, the denoised medical image. The machine-learning method may therefore be trained to generate a clean or denoised image from a (noisy) medical image.

In some examples, the machine-learning method may only output an uncalibrated denoised medical image. The uncalibrated denoised medical image may be then be (re)calibrated using the noise map. In particular, the uncalibrated denoised medical image may be modified to perform the inverse of the procedure executed in step 130, using the uncalibrated denoised medical image and the noise map, to produce the denoised medical image.

By way of example, if step 130 comprises performing a pixel-wise division of the medical image by the noise map, then the uncalibrated denoised medical image may undergo a pixel-wise multiplication with the noise map.

(Re)calibration may not be required in some circumstances or embodiments, for instance, if step 130 comprises decorrelating noise within the medical image.

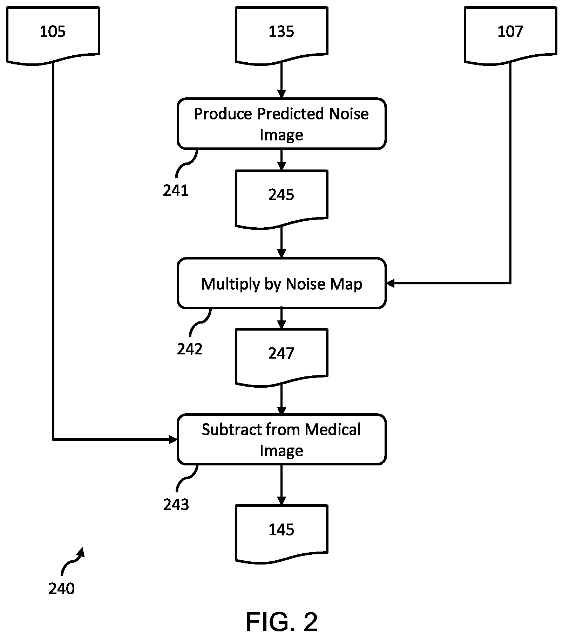

Figure 2 illustrates another embodiment for performing process 140, which has been labelled process 240 for the sake of distinction.

The process 240 comprises a step 241 of inputting the modified medical image 135 to a machine-learning method configured to generate a predicted noise image 245. The predicted noise image is an image that contains a same number of pixels as the medical image, and indicates (for each pixel of the medical image) an estimated/predicted measure of noise of that pixel.

The process 240 then performs a step 242 of multiplying the predicted noise image 245 by the noise map, i.e. to renormalize the estimated noise image 245, to thereby produce a calibrated predicted noise image 247.

Of course, if the medical image is not divided by the noise map in step 130, then step 242 may be modified to perform the inverse to the procedure executed in step 130 using the estimated noise image in place of the modified noise image (i.e. so that the inverse of procedure 130 is performed in step 242, using the estimated noise image 245 and the noise map 107 as inputs). Thus, a reversal to (or inverse of) the modification performed on the medical image to produce the modified medical image may be applied to the estimated noise image to calibrate the estimated noise image.

The process 240 then subtracts in a step 243 the calibrated predicted noise image from the medical image 105 to produce the denoised medical image 145.

In some examples, the calibrated predicted noise image is weighted (e.g. scaled down) before being subtracted from the medical image. This approach recognizes that some clinicians would prefer to retain some (non-zero) level of noise in the medical image to reduce an artificial appearance of the denoised medical image, which might otherwise distract the clinician.

Thus, step 243 may comprise subtracting a scaled version of the calibrated predicted noise image from the medical image. The scaled version may be calculated by multiplying the value of each pixel of the calibrated predicted noise image by a predetermined value, wherein the predetermined value is between 0 and 1, e.g. between 0.25 and 0.75.

In this way, for process 240, the machine-learning method is configured to receive, as input, the modified medical image and provide, as output, the predicted noise image. The predicted noise image indicates, for each pixel, a predicted or inferred amount of noise at that pixel. This may be on a pixel-by-pixel basis.

In the proposed approaches, each machine-learning method processes medical images that have already been normalized or homogenized (e.g. decorrelated) based on statistical information about noise. This means that the images provided as input to the machine-learning method are already at consistent and/or decorrelated noise levels. The machine-learning method may thereby be trained on normalized medical images, reducing the risk of overfitting (e.g. to a particular reconstruction filter or noise level).

Turning back to Figure 1, the method 100 may further comprise a step 150 of controlling a user interface to provide a visual representation of the denoised medical image 145 output by process 140. The user interface may be a display, such as a monitor or the like.

In some examples, the method 100 may comprise a step 155 of storing the denoised medical image, e.g. in a memory or storage unit. Step 155 may comprise storing the denoised medical image in an electronic medical record of the subject of the denoised medical image.

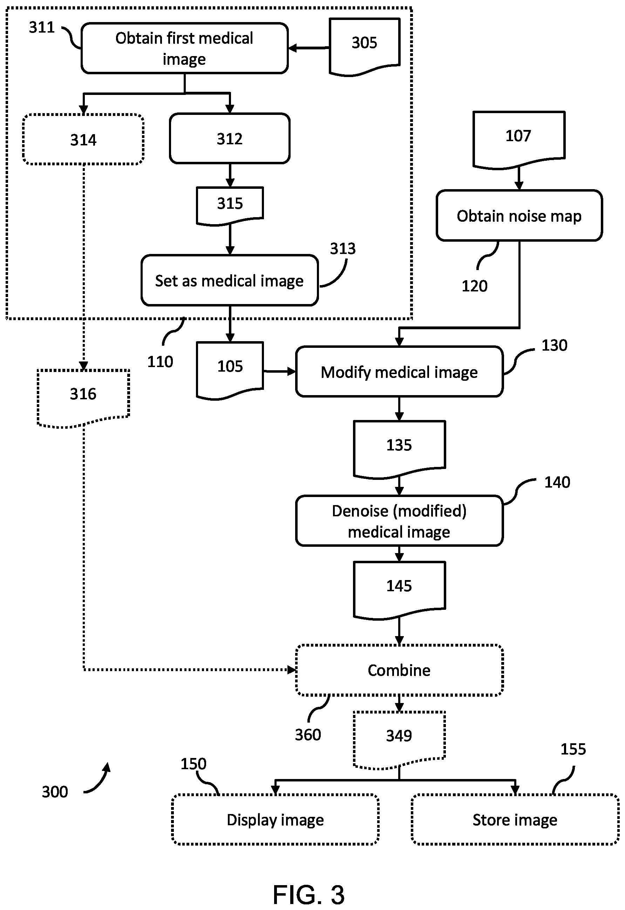

Figure 3 illustrates a method 300 according to another embodiment.

Method 300 differs from method 100 previously described in that the step 110 of obtaining the medical image comprises: a step 311 of obtaining a first medical image 305; a step 312 of processing the first medical image using a frequency filter (that filters according to a predetermined frequency range) to obtain a first filtered medical image 315; and a step 313 of setting the first filtered medical image as the medical image. The frequency filter may, for instance, be a high-pass or a bandpass filter.

In this way, the medical image that undergoes denoising may be a frequency filtered part of a medical image.

Preferably, the frequency filtered part is a high frequency part of the medical image, i.e. a part of the medical image having a frequency greater than a predetermined frequency value. It is herein recognized that noise in a medical image may usually be high-frequency, such that more efficient and improved denoising can be performed by denoising only the high-frequency part of the medical image.

In some examples, the step 110 further comprises a step 314 of processing the first medical image using another frequency filter to obtain a second filtered medical image 316. The second filtered medical image has a different frequency range to the first filtered medical image. In one example, the second filtered medical image may be the part of the (original) medical image that is not in the predetermined frequency range.

For instance, if the frequency filter is a high-pass filter, the second filtered medical image may be a low-pass filtered part of the medical image, i.e. the medical image is processed using a low-pass filter. The low-pass filter and the high-pass filter may have a same cut-off frequency, so that a medical image is effectively split (by steps 312 and 314) into high and low frequency medical images, forming the first and second filtered medical images respectively.

An alternative to optional step 314 for producing the second filtered medical image 316 is to subtract the first filtered medical image 315 from the first medical image 305.

Similarly, if step 314 comprises processing the first medical image using a filter, such as a low-pass filter, step 312 could be modified to instead comprise subtracting the second filtered medical image 316 from the first medical image 305 to produce the first filtered medical image 315.

The method 300 may further comprise a step 360 of combining the second filtered medical image 316 and the denoised medical image 145 output by process 140 to reform a denoised version of the first medical image 349. Step 360 may comprise simply summing the second filtered and the denoised medical image output by process 140.

Like method 100, method 300 may comprise a step 150 of controlling a user interface to provide a visual representation of the denoised medical image 145 output by process 140, and/or a step 155 of storing the denoised medical image, e.g. in a memory or storage unit. Method 300 may be adapted accordingly.

It has been identified the proposed approaches are particularly effective when the machine-learning method is a residual learning method. A residual learning method produces, as output, a predicted noise image that contains a same number of pixels as the medical image, and provides (for each pixel) a measure of predicted noise for that pixel.

Testing and analysis of the herein proposed approach has shown that the proposed approach generalizes well to different reconstruction filters as well as noise levels higher than those seen in during training of the machine-learning method, such as those resulting from use of lower radiation dose levels. The herein proposed approach therefore increases machine-learning robustness in practice.

It is recognized that, for some methods of generating a noise map, the noise map that is actually computed may not reflect the statistical variations of noise levels introduced by different reconstruction filters. Rather, they may reflect difference in noise levels due to other factors, such as pervasive system errors, use of lower radiation doses or the like.

In this case, to generalize the proposed approach for different systems, the noise map obtained in step 120 of any previously described method can simply be scaled by a global scaling factor, before being used to process the medical image.

For instance, in the field of CT imaging, it is well known that a reconstruction filter commonly comprises two parts or functions, namely the ramp and an additional modulation transfer function (MTF). A more complete understanding is set out in Thorsten M. Buzug (2008). Computed Tomography. Springer-Verlag Berlin Heidelberg. Generally, different reconstruction filters used in CT imaging differ only by the MTF modulation part.

This global scaling factor can be computed from the area under (Ramp * MTF)² curve for the reconstruction filter used, where MTF is the modulation transfer function. Indeed, it is considered that noise variance will scale linearly with the area under (Ramp * MTF)². The (square root of the) ratio between the area for the reconstruction filter used to reconstruct the medical image being processed, and the corresponding area for the reconstruction filter used in the training of the machine-learning method provides an adequate scaling factor for the noise map.

Scaling of the noise map is not required, for instance, if the generation of the noise map is image-based, or otherwise takes a reconstruction filter (used to generate the medical image) into account.

In summary, this approach allows the use of pre-trained machine-learning methods for application on unseen reconstruction filters, by scaling the corresponding standard deviation noise map by the above described scaling factor. Since this factor is only a function of two filters, this comes at virtually no additional computational cost or overhead, and thus does not require re-training of the machine-learning method.

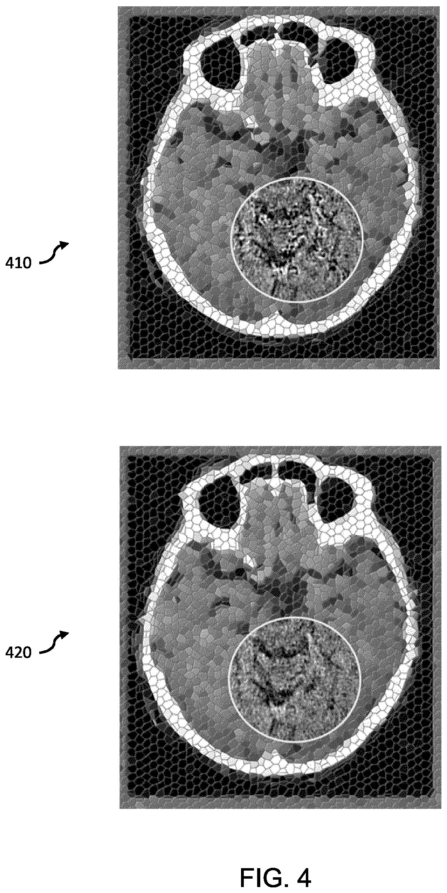

Figure 4 illustrates the effect of the proposed concepts on denoising a medical image. Figure 4 provides two denoised CT head images, which have been partially obscured (outside of the area bounded by the white circle on each image). Each CT image has been produced by processing a same medical CT image obtained with a 25% dose level.

A first denoised CT head image 410 has been produced using a conventional denoising machine-learning method (specifically a convolutional neural network), and does not make use of any normalization or modification of the medical image using a noise map, such as proposed in the present invention.

A second denoised CT head image 420 has been produced using the proposed denoising approach, specifically with normalization of the statistical parameter of the noise before being processed using a machine-learning method.

In both cases, the machine-learning method has been trained using CT images obtained at a 25% dose level and reconstructed using a first reconstruction filter. The medical CT image which is then denoised (to produce head image 420) was produced using a second, different reconstruction filter with less noise suppression. Although different reconstruction filters result in different noise characteristics, they may also provide other benefits, e.g. providing sharper or smoother images and/or emphasizing different anatomical features. An operator may therefore select a noisier or less noisy reconstruction filter depending upon their clinical preference.

It can clearly be seen that using the proposed approach produces a denoised image with less noise, thereby making the approach more robust to reconstruction filters with higher noise levels than seen during the training of the machine-learning method used in the denoising method.

It is noted that the noise map obtained during the production of the second denoised CT head image was scaled by 1.4 to more accurately reflect the higher noise level in the image as a result of the different reconstruction filter, following the approach previously described. The value of 1.4 was selected based upon an above-described mechanism for determining the scaling factor.

Proposed embodiments make use of a machine-learning method. A machine-learning method is any self-training algorithm that processes input data in order to produce or predict output data. Here, the input data comprises a modified medical image and the output data comprises either a denoised medical image or a predicted noise image.

Suitable machine-learning methods for being employed in the present invention will be apparent to the skilled person. Examples of suitable machine-learning methods include decision tree algorithms and artificial neural networks. Other machine-learning methods such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

For the present disclosure, embodiments are particularly advantageous when a residual learning machine-learning method is used, e.g. a residual neural network. A residual neural network differs from a conventional neural network in that skip connections can be used, e.g. so that outputs of one layer may skip one or more layers (i.e. not all outputs of any given layer need to be provided as input to a sequentially next layer).

Methods of training a machine-learning method are well known. Typically, such methods comprise obtaining a training dataset, comprising training input data entries and corresponding training output data entries. An initialized machine-learning method is applied to each input data entry to generate predicted output data entries. An error between the predicted output data entries and corresponding training output data entries is used to modify the machine-learning method. This process can be repeated until the error converges, and the predicted output data entries are sufficiently similar (e.g. ±1%) to the training output data entries. This is commonly known as a supervised learning technique.

For example, where the machine-learning method is formed from a neural network, (weightings of) the mathematical operation of each neuron may be modified until the error converges. Known methods of modifying a neural network include gradient descent, backpropagation algorithms and so on.

The training input data entries correspond to example modified medical images. In particular, each training input data entry should contain a medical image that has been pre-processed or modified using a noise map (for that medical image), e.g. have been subject to step 130 as described with reference to method 100. This would improve a reliability and accuracy of the machine-learning method when employed in method 100.

The training output data entries correspond to example denoised medical images and/or noise images. Information on the reconstruction filter used to generate the example medical images for the training input data entries may be stored, e.g. to facilitate scaling of a noise map as previously described.

The training input data entries may, for instance, be (modified) medical images that have had artificial noise added, with the training output data entries being the (modified) medical images to which no artificial noise has been added.

Any herein proposed method may be performed by the imaging system itself (i.e. the system that generates the medical image(s), a processing system on the same premises as the imaging system, on a mobile device (e.g. a smart phone, tablet or laptop) or using a distributed processing system, i.e. "the cloud".

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

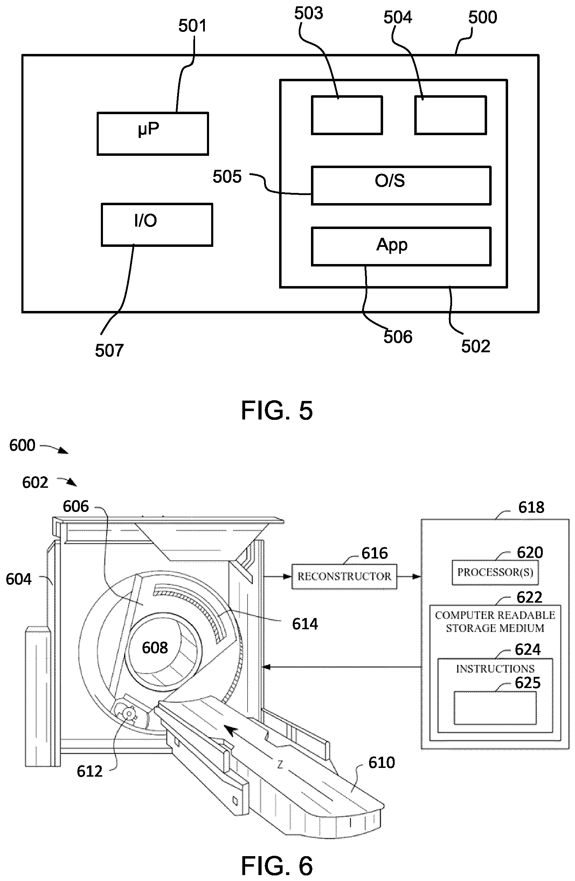

By way of further example, Figure 5 illustrates an example of a processing system 500 within which one or more parts of an embodiment may be employed. Various operations discussed above may utilize the capabilities of the processing system 500. For example, one or more parts of a system for denoising a medical image may be incorporated in any element, module, application, and/or component discussed herein. In this regard, it is to be understood that system functional blocks can run on a single computer or may be distributed over several computers and locations (e.g. connected via internet).

The processing system 500 includes, but is not limited to, PCs, workstations, laptops, PDAs, palm devices, servers, storages, and the like. Generally, in terms of hardware architecture, the processing system 500 may include one or more processors 501, memory 502, and one or more I/O devices 507 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 501 is a hardware device for executing software that can be stored in the memory 502. The processor 501 can be virtually any custom made or commercially available processor, a central processing unit (CPU), a digital signal processor (DSP), or an auxiliary processor among several processors associated with the processing system 500, and the processor 501 may be a semiconductor based microprocessor (in the form of a microchip) or a microprocessor.

The memory 502 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 502 may incorporate electronic, magnetic, optical, and/or other types of storage media. Note that the memory 502 can have a distributed architecture, where various components are situated remote from one another, but can be accessed by the processor 501.

The software in the memory 502 may include one or more separate programs, each of which comprises an ordered listing of executable instructions for implementing logical functions. The software in the memory 502 includes a suitable operating system (O/S) 505, compiler 504, source code 503, and one or more applications 506 in accordance with exemplary embodiments. As illustrated, the application 506 comprises numerous functional components for implementing the features and operations of the exemplary embodiments. The application 506 of the processing system 500 may represent various applications, computational units, logic, functional units, processes, operations, virtual entities, and/or modules in accordance with exemplary embodiments, but the application 506 is not meant to be a limitation.

The operating system 505 controls the execution of other computer programs, and provides scheduling, input-output control, file and data management, memory management, and communication control and related services. It is contemplated by the inventors that the application 506 for implementing exemplary embodiments may be applicable on all commercially available operating systems.

Application 506 may be a source program, executable program (object code), script, or any other entity comprising a set of instructions to be performed. When a source program, then the program is usually translated via a compiler (such as the compiler 504), assembler, interpreter, or the like, which may or may not be included within the memory 502, so as to operate properly in connection with the O/S 505. Furthermore, the application 506 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 507 may include input devices such as, for example but not limited to, a mouse, keyboard, scanner, microphone, camera, etc. Furthermore, the I/O devices 507 may also include output devices, for example but not limited to a printer, display, etc. Finally, the I/O devices 507 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 507 also include components for communicating over various networks, such as the Internet or intranet.

If the processing system 500 is a PC, workstation, intelligent device or the like, the software in the memory 502 may further include a basic input output system (BIOS) (omitted for simplicity). The BIOS is a set of essential software routines that initialize and test hardware at startup, start the O/S 505, and support the transfer of data among the hardware devices. The BIOS is stored in some type of read-only-memory, such as ROM, PROM, EPROM, EEPROM or the like, so that the BIOS can be executed when the processing system 500 is activated.

When the processing system 500 is in operation, the processor 501 is configured to execute software stored within the memory 502, to communicate data to and from the memory 502, and to generally control operations of the processing system 500 pursuant to the software. The application 506 and the O/S 505 are read, in whole or in part, by the processor 501, perhaps buffered within the processor 501, and then executed.

When the application 506 is implemented in software it should be noted that the application 506 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 506 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

Figure 6 schematically illustrates a system 600 including an imaging system 602 and a processing system 612. Here, the imaging system is a CT scanner configured for spectral (multi-energy) imaging. However, other forms of imaging system could be used. The processing system 612 may be embodied as the processing system 500 previously described.

The illustrated imaging system 602 includes a generally stationary gantry 604 and a rotating gantry 606, which is rotatably supported by the stationary gantry 604 and rotates around an examination region 608 about a z-axis. A subject support 610, such as a couch, supports an object or subject in the examination region 608.

A radiation source 612, such as an x-ray tube, is rotatably supported by the rotating gantry 606, rotates with the rotating gantry 606, and emits radiation that traverses the examination region 608. In one instance, the radiation source 612 includes a single broad spectrum x-ray tube. In another instance, the radiation source 612 includes a single x-ray tube configured to switch between at least two different emission voltages (e.g., 80 kVp and 640 kVp) during scanning. In yet another instance, the radiation source 612 includes two or more x-ray tubes configured to emit radiation having different mean spectra. In still another instance, the radiation source 612 includes a combination thereof.

A radiation sensitive detector array 614 subtends an angular arc opposite the radiation source 612 across the examination region 608. The radiation sensitive detector array 614 detects radiation traversing the examination region 608 and generates an electrical signal(s) (projection data) indicative thereof. Where the radiation source 612 includes a single broad spectrum x-ray tube, the radiation sensitive detector array 612 includes energy-resolving detectors (e.g., direct conversion photon counting detectors, at least two sets of scintillators with different spectral sensitivities (multi-layer), etc.). With kVp switching and multi-tube configurations, the detector array 614 can include single layer detectors, direct conversion photon counting detectors, and/or multi-layer detectors. The direct conversion photon counting detectors may include a conversion material such as CdTe, CdZnTe, Si, Ge, GaAs, or other direct conversion material. An example of multi-layer detector includes a double decker detector such as the double decker detector described in US patent 7,968,853 B2, filed April 60, 2006, and entitled "Double Decker Detector for Spectral CT."

A reconstructor 616 receives spectral projection data from the detector array 614 and reconstructs spectral volumetric image data such as sCCTA image data, a high-energy image, a low energy image, a photoelectric image, a Compton scatter image, an iodine image, a calcium image, a virtual non-contrast image, a bone image, a soft tissue image, and/or other basis material image. The reconstructor 616 can also reconstruct non-spectral volumetric image data, e.g., by combining spectral projection data and/or spectral volumetric image data. Generally, the spectral projection data and/or spectral volumetric image data will include data for at least two different energies and/or energy ranges.

In this way, the reconstructor 616 generates or reconstructs a medical image.

The processing system 618 here serves as an operator console. The console 618 includes a human readable output device such as a monitor and an input device such as a keyboard, mouse, etc. Software resident on the console 618 allows the operator to interact with and/or operate the scanner 602 via a graphical user interface (GUI) or otherwise. The console 618 further includes a processor 620 (e.g., a microprocessor, a controller, a central processing unit, etc.) and a computer readable storage medium 622, which excludes non-transitory medium, and includes transitory medium such as a physical memory device, etc. The computer readable storage medium 622 includes instructions 624 for denoising a produced medical image, i.e. contains a medical image denoiser 625. The processor 620 is configured to execute the instructions 624. The processor 620 may additionally be configured to execute one or more computer readable instructions carried by a carrier wave, a signal and/or other transitory medium. In a variation, the processor 620 and the computer readable storage medium 622 are part of another processing system, which is separate from the processing system 618.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (100) of denoising a medical image to thereby produce a denoised medical image, the computer-implemented method comprising:
obtaining the medical image (110) formed of a plurality of pixels;
obtaining a noise map (120) containing an estimated measure of a statistical parameter for each pixel of the medical image;
modifying the medical image (130) using the noise map to produce a modified medical image; and
processing the modified medical image (140), using a machine-learning method, to produce the denoised medical image; **characterised in that** the step of modifying the medical image comprises dividing the medical image by the noise map.

2. The computer-implemented method of claim 1, wherein the step of processing the modified medical image comprises:
processing the modified medical image using the machine-learning method to generate a predicted noise image, the predicted noise image representing a predicted amount of noise in each pixel of the modified medical image;
multiplying the modified medical image by the noise map to produce a calibrated predicted noise image; and
subtracting the calibrated predicted noise image or a scaled version of the calibrated predicted noise image from the medical image to produce the denoised medical image.

3. The computer-implemented method of claim 1, wherein the step of processing the modified medical image comprises inputting the modified medical image to the machine-learning method and receiving, as output from the machine-learning method, the denoised medical image.

4. The computer-implemented method of claim 1, wherein the machine-learning method comprises a neural network.

5. The computer-implemented method of claim 1, wherein the noise map provides an estimated amount of standard deviation or variance of noise for each pixel of the medical image.

6. The computer-implemented method of claim 1, wherein the noise map provides, for each pixel of the medical image, an estimated correlation between the noise of said pixel and the noise of one or more neighboring pixels.

7. The computer-implemented method of claim 1, wherein:
the medical image is one of a plurality of medical images, that represent a same scene, produced by a multi-channel imaging process; and
the noise map provides, for each pixel of the medical image, an estimated measure of a covariance or correlation between the noise of that pixel and the noise of a corresponding pixel of another of the plurality of medical images.

8. The computer-implemented method of claim 1, wherein the step of obtaining the medical image comprises:
obtaining a first medical image;
processing the first medical image using a frequency filter to obtain a first filtered medical image having values within a predetermined frequency range; and
setting the first filtered medical image as the medical image.

9. The computer-implemented method of claim 8, further comprising:
processing the first medical image to obtain a second filtered medical image having values within a second, different predetermined frequency range; and
combining the second filtered medical image and the denoised medical image to produce a denoised first medical image.

10. The computer-implemented method of claim 1, wherein:
the medical image is a medical image that has been reconstructed from raw data using a first reconstruction algorithm; and
the machine-learning method is a machine-learning method that has been trained using a training dataset that includes one or more training images that have been reconstructed from raw data using a second, different reconstruction algorithm.

11. The computer-implemented method of claim 1, wherein the medical image is a computed tomography medical image.

12. A computer program product comprising computer program code means which, when executed on a computing device having a processing system, cause the processing system to perform all of the steps of the method according to claim 1.

13. A processing system (500) configured to denoise a medical image to thereby produce a denoised medical image, the processing system being configured to:
obtain the medical image (110) formed of a plurality of pixels;
obtain a noise map (120) containing an estimated measure of a statistical parameter for each pixel of the medical image;
modify the medical image (130) using the noise map to produce a modified medical image; and
process the modified medical image (140), using a machine-learning method, to produce the denoised medical image;
**characterised in that** the step of modifying the medical image comprises dividing the medical image by the noise map.

14. A system (600) comprising:
the processing system of claim 13; and
a medical imaging system configured to generate the medical image and provide the generated medical image to the processing system.

## Patentansprüche

1. Computerimplementiertes Verfahren (100) zum Entrauschen eines medizinischen Bildes, um dadurch ein entrauschtes medizinisches Bild zu erzeugen, wobei das computerimplementierte Verfahren Folgendes umfasst:
Erhalten des medizinischen Bildes (110), das aus einer Vielzahl von Pixeln gebildet ist;
Erhalten einer Rauschkarte (120), die ein geschätztes Maß eines statistischen Parameters für jedes Pixel des medizinischen Bildes enthält;
Modifizieren des medizinischen Bildes (130) unter Verwendung der Rauschkarte, um ein modifiziertes medizinisches Bild zu erzeugen; und
Verarbeiten des modifizierten medizinischen Bildes (140) unter Verwendung eines maschinellen Lernverfahrens, um das entrauschte medizinische Bild zu erzeugen; **dadurch gekennzeichnet, dass**
der Schritt des Modifizierens des medizinischen Bildes Folgendes umfasst
Teilen des medizinischen Bildes durch die Rauschkarte.

2. Computerimplementiertes Verfahren nach Anspruch 1, wobei der Schritt des Verarbeitens des modifizierten medizinischen Bildes Folgendes umfasst:
Verarbeiten des modifizierten medizinischen Bildes unter Verwendung des maschinellen Lernverfahrens, um ein vorhergesagtes Rauschbild zu erzeugen, wobei das vorhergesagte Rauschbild eine vorhergesagte Rauschmenge in jedem Pixel des modifizierten medizinischen Bildes darstellt;
Multiplizieren des modifizierten medizinischen Bildes mit der Rauschkarte, um ein kalibriertes vorhergesagtes Rauschbild zu erzeugen; und
Subtrahieren des kalibrierten vorhergesagten Rauschbildes oder einer skalierten Version des kalibrierten vorhergesagten Rauschbildes von dem medizinischen Bild, um das entrauschte medizinische Bild zu erzeugen.

3. Computerimplementiertes Verfahren nach Anspruch 1, wobei der Schritt des Verarbeitens des modifizierten medizinischen Bildes Eingeben des modifizierten medizinischen Bildes in das maschinelle Lernverfahren und Empfangen des entrauschten medizinischen Bildes als Ausgabe des maschinellen Lernverfahrens umfasst.

4. Computerimplementiertes Verfahren nach Anspruch 1, wobei das maschinelle Lernverfahren ein neuronales Netz umfasst.

5. Computerimplementiertes Verfahren nach Anspruch 1, wobei die Rauschkarte einen geschätzten Betrag einer Standardabweichung oder Varianz des Rauschens für jedes Pixel des medizinischen Bildes bereitstellt.

6. Computerimplementiertes Verfahren nach Anspruch 1, wobei die Rauschkarte für jedes Pixel des medizinischen Bildes eine geschätzte Korrelation zwischen dem Rauschen des Pixels und dem Rauschen eines oder mehrerer benachbarter Pixel bereitstellt.

7. Computerimplementiertes Verfahren nach Anspruch 1, wobei:
das medizinische Bild eines von einer Vielzahl von medizinischen Bildern ist, die eine gleiche Szene darstellen und durch einen Mehrkanal-Bildgebungsprozess erzeugt werden; und
die Rauschkarte für jedes Pixel des medizinischen Bildes ein geschätztes Maß einer Kovarianz oder Korrelation zwischen dem Rauschen dieses Pixels und dem Rauschen eines entsprechenden Pixels eines anderen der Vielzahl von medizinischen Bildern bereitstellt.

8. Computerimplementiertes Verfahren nach Anspruch 1, wobei der Schritt des Erhaltens des medizinischen Bildes Folgendes umfasst:
Erhalten eines ersten medizinischen Bildes;
Verarbeiten des ersten medizinischen Bildes unter Verwendung eines Frequenzfilters, um ein erstes gefiltertes medizinisches Bild zu erhalten, das Werte innerhalb eines vorbestimmten Frequenzbereichs aufweist; und
Festlegen des ersten gefilterten medizinischen Bildes als medizinisches Bild.

9. Computerimplementiertes Verfahren nach Anspruch 8, das weiter Folgendes umfasst:
Verarbeiten des ersten medizinischen Bildes, um ein zweites gefiltertes medizinisches Bild zu erhalten, das Werte innerhalb eines zweiten, unterschiedlichen vorbestimmten Frequenzbereichs aufweist; und
Kombinieren des zweiten gefilterten medizinischen Bildes und des entrauschten medizinischen Bildes, um ein entrauschtes erstes medizinisches Bild zu erzeugen.

10. Computerimplementiertes Verfahren nach Anspruch 1, wobei:
das medizinische Bild ein medizinisches Bild ist, das aus Rohdaten unter Verwendung eines ersten Rekonstruktionsalgorithmus rekonstruiert wurde; und
das maschinelle Lernverfahren ein maschinelles Lernverfahren ist, das unter Verwendung eines Trainingsdatensatzes trainiert wurde, der ein oder mehrere Trainingsbilder beinhaltet, die unter Verwendung eines zweiten, unterschiedlichen Rekonstruktionsalgorithmus aus Rohdaten rekonstruiert wurden.

11. Computerimplementiertes Verfahren nach Anspruch 1, wobei das medizinische Bild ein computertomographisches medizinisches Bild ist.

12. Computerprogrammprodukt, das Computerprogrammcodemittel umfasst, die, wenn sie auf einer Rechenvorrichtung ausgeführt werden, die ein Verarbeitungssystem aufweist, das Verarbeitungssystem veranlassen, alle Schritte des Verfahrens nach Anspruch 1 durchzuführen.

13. Verarbeitungssystem (500), das dazu konfiguriert ist, ein medizinisches Bild zu entrauschen, um dadurch ein entrauschtes medizinisches Bild zu erzeugen, wobei das Verarbeitungssystem dazu konfiguriert ist:
das aus einer Vielzahl von Pixeln gebildete medizinische Bild (110) zu erhalten;
eine Rauschkarte (120) zu erhalten, die ein geschätztes Maß eines statistischen Parameters für jedes Pixel des medizinischen Bildes enthält;
das medizinische Bild (130) unter Verwendung der Rauschkarte zu modifizieren, um ein modifiziertes medizinisches Bild zu erzeugen; und
das modifizierte medizinische Bild (140) unter Verwendung eines maschinellen Lernverfahrens zu verarbeiten, um das entrauschte medizinische Bild zu erzeugen;
**dadurch gekennzeichnet, dass**
der Schritt des Modifizierens des medizinischen Bildes das Teilen des medizinischen Bildes durch die Rauschkarte umfasst.

14. System (600), umfassend:
das Verarbeitungssystem nach Anspruch 13; und
ein medizinisches Bildgebungssystem, das dazu konfiguriert ist, das medizinische Bild zu erzeugen und das erzeugte medizinische Bild dem Verarbeitungssystem bereitzustellen.

## Revendications

1. Procédé (100) mis en œuvre par ordinateur de débruitage d'une image médicale pour produire, de ce fait, une image médicale débruitée, le procédé mis en œuvre par ordinateur comprenant :
l'obtention de l'image médicale (110) formée d'une pluralité de pixels ;
l'obtention d'une carte de bruit (120) contenant une mesure estimée d'un paramètre statistique pour chaque pixel de l'image médicale ;
la modification de l'image médicale (130) à l'aide de la carte de bruit pour produire une image médicale modifiée ; et
le traitement de l'image médicale modifiée (140), à l'aide d'un procédé d'apprentissage automatique, pour produire l'image médicale débruitée ; **caractérisé en ce que**
l'étape de modification de l'image médicale comprend
la division de l'image médicale par la carte de bruit.

2. Procédé mis en œuvre par ordinateur selon la revendication 1, dans lequel l'étape de traitement de l'image médicale modifiée comprend :
le traitement de l'image médicale modifiée à l'aide du procédé d'apprentissage automatique pour générer une image de bruit prédite, l'image de bruit prédite représentant une quantité prédite de bruit dans chaque pixel de l'image médicale modifiée ;
la multiplication de l'image médicale modifiée par la carte de bruit pour produire une image de bruit prédite étalonnée ; et
la soustraction de l'image de bruit prédite étalonnée, ou une version mise à l'échelle de l'image de bruit prédite étalonnée, de l'image médicale pour produire l'image médicale débruitée.

3. Procédé mis en œuvre par ordinateur selon la revendication 1, dans lequel l'étape de traitement de l'image médicale modifiée comprend l'entrée de l'image médicale modifiée dans le procédé d'apprentissage automatique et la réception, comme sortie provenant du procédé d'apprentissage automatique, de l'image médicale débruitée.

4. Procédé mis en œuvre par ordinateur selon la revendication 1, dans lequel le procédé d'apprentissage automatique comprend un réseau neuronal.

5. Procédé mis en œuvre par ordinateur selon la revendication 1, dans lequel la carte de bruit fournit une quantité estimée d'écart type ou de variance de bruit pour chaque pixel de l'image médicale.

6. Procédé mis en œuvre par ordinateur selon la revendication 1, dans lequel la carte de bruit fournit, pour chaque pixel de l'image médicale, une corrélation estimée entre le bruit dudit pixel et le bruit d'un ou de plusieurs pixels voisins.

7. Procédé mis en œuvre par ordinateur selon la revendication 1, dans lequel :
l'image médicale est une image médicale d'une pluralité d'images médicales, qui représentent une même scène, produites par un processus d'imagerie multicanal ; et
la carte de bruit fournit, pour chaque pixel de l'image médicale, une mesure estimée d'une covariance ou d'une corrélation entre le bruit de ce pixel et le bruit d'un pixel correspondant d'une autre image médicale de la pluralité d'images médicales.

8. Procédé mis en œuvre par ordinateur selon la revendication 1, dans lequel l'étape d'obtention de l'image médicale comprend :
l'obtention d'une première image médicale ;
le traitement de la première image médicale à l'aide d'un filtre de fréquence pour obtenir une première image médicale filtrée présentant des valeurs dans une plage de fréquences prédéterminée ; et
la définition de la première image médicale filtrée en tant qu'image médicale.

9. Procédé mis en œuvre par ordinateur selon la revendication 8, comprenant en outre :
le traitement de la première image médicale pour obtenir une seconde image médicale filtrée présentant des valeurs dans une seconde plage de fréquences prédéterminée différente ; et
la combinaison de la seconde image médicale filtrée et de l'image médicale débruitée pour produire une première image médicale débruitée.

10. Procédé mis en œuvre par ordinateur selon la revendication 1, dans lequel :
l'image médicale est une image médicale qui a été reconstruite à partir de données brutes à l'aide d'un premier algorithme de reconstruction ; et
le procédé d'apprentissage automatique est un procédé d'apprentissage automatique qui a été entraîné à l'aide d'un ensemble de données d'entraînement qui inclut une ou plusieurs images d'entraînement qui ont été reconstruites à partir de données brutes à l'aide d'un second algorithme de reconstruction différent.

11. Procédé mis en œuvre par ordinateur selon la revendication 1, dans lequel l'image médicale est une image médicale de tomodensitométrie.

12. Produit de programme informatique comprenant des moyens de code de programme informatique qui, lorsqu'ils sont exécutés sur un dispositif informatique présentant un système de traitement, amènent le système de traitement à mettre en œuvre toutes les étapes du procédé selon la revendication 1.

13. Système de traitement (500) configuré pour débruiter une image médicale pour produire, de ce fait, une image médicale débruitée, le système de traitement étant configuré pour :
obtenir l'image médicale (110) formée d'une pluralité de pixels ;
obtenir une carte de bruit (120) contenant une mesure estimée d'un paramètre statistique pour chaque pixel de l'image médicale ;
modifier l'image médicale (130) à l'aide de la carte de bruit pour produire une image médicale modifiée ; et
traiter l'image médicale modifiée (140), à l'aide d'un procédé d'apprentissage automatique, pour produire l'image médicale débruitée ;
**caractérisé en ce que**
l'étape de modification de l'image médicale comprend la division de l'image médicale par la carte de bruit.

14. Système (600), comprenant :
le système de traitement selon la revendication 13 ; et
un système d'imagerie médicale configuré pour générer l'image médicale et fournir l'image médicale générée au système de traitement.
